(19) Europäisches Patentamt
European Patent Office
Office européen des brevets

(11) Numéro de publication : **0 021 926**
**B1**

(12) **FASCICULE DE BREVET EUROPÉEN**

(45) Date de publication du fascicule du brevet : **19.05.82**

(51) Int. Cl.³ : **C 07 C 49/707**, C 07 C 45/67// C07C69/743

(21) Numéro de dépôt : **80400810.0**

(22) Date de dépôt : **06.06.80**

(54) **Procédé de préparation d'alléthrolone optiquement active.**

(30) Priorité : **12.06.79 FR 7914978**

(43) Date de publication de la demande : **07.01.81 (Bulletin 81/01)**

(45) Mention de la délivrance du brevet : **19.05.82 Bulletin 82/20**

(84) Etats contractants désignés : **CH DE GB IT LI NL**

(56) Documents cités :
**FR - A - 2 223 347**
**FR - A - 2 362 830**

(73) Titulaire : **ROUSSEL-UCLAF**
**35, boulevard des Invalides**
**F-75007 Paris (FR)**

(72) Inventeur : **Martel, Jacques**
**15, rue Douvillez**
**F-93140 Bondy (FR)**
Inventeur : **Tessier, Jean**
**30, rue Jean Moulin**
**F-94300 Vincennes (FR)**
Inventeur : **Demoute, Jean-Pierre**
**249 bis rue de Rosny**
**F-93100 Montreuil S/Bois (FR)**

(74) Mandataire : **Fritel, Hubert et al**
**102, route de Noisy Boîte Postale No 9**
**F-93230 Romainville (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Procédé de préparation d'alléthrolone optiquement active

La présente invention concerne un procédé de préparation d'alléthrolone optiquement active par scission d'un ester d'acide chiral d'alléthrolone optiquement active à l'aide d'un halogénure de bore.

L'invention a pour objet un procédé de préparation de (R) ou (S) 2-allyl 3-méthyl 4-hydroxy 2-cyclopenten-1-one, caractérisé en ce l'on fait réagir un halogénure de bore sur un ester d'acide carboxylique chiral 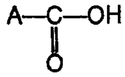 et de (R) ou de (S) 2-allyl 3-méthyl 4-hydroxy 2-cyclopenten-1-one, de formule (I) :

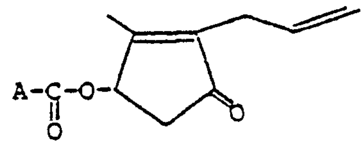

(I)

dans laquelle A représente le reste organique de l'acide chiral carboxylique 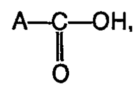, soumet le mélange réactionnel résultant à l'action de l'eau pour obtenir, avec rétention de configuration par rapport à celle existant dans l'ester de départ, respectivement la (R) ou la (S) 2-allyl 3-méthyl 4-hydroxy 2-cyclopenten-1-one et l'acide chiral.

Dans le procédé selon l'invention, l'acide chiral 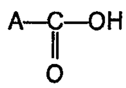 est notamment un acide aliphatique ou un acide qui comporte un ou plusieurs cycles, tel un acide de structure polycyclanique, un acide de structure stéroïde ou un acide cyclopropane carboxylique.

L'invention a plus particulièrement pour objet un procédé tel que défini précédemment, dans lequel l'acide chiral 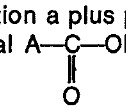 est un acide carboxylique choisi dans le groupe constitué par les acides chiraux dont les noms suivent :

— les acides 2,2-diméthyl 3-(2-méthyl 1-propényl) cyclopropane-1-carboxyliques,
— les acides 2,2-diméthyl 3-(2,2-dibromovinyl) cyclopropane-1-carboxyliques,
— les acides 2,2-diméthyl 3-(2,2-dichlorovinyl) cyclopropane-1-carboxyliques,
— les acides 2,2-diméthyl 3-(cyclobutylidène méthyl) cyclopropane-1-carboxyliques,
— les acides 2,2-diméthyl 3-(cyclopentylidène méthyl) cyclopropane-1-carboxyliques,
— les acides 2,2-diméthyl 3-(fluorénylidène méthyl) cyclopropane-1-carboxyliques,
— les acides 2,2-diméthyl 3-(2-oxo 3-oxa cyclopentylidène méthyl) cyclopropane-1-carboxyliques,
— les acides 2,2-diméthyl 3-(2,2-difluorovinyl) cyclopropane-1-carboxyliques,
— les acides 2-(3-chlorophényl)2-isopropyl acétiques.

L'invention a notamment pour objet un procédé tel que défini précédemment, caractérisé en ce que l'acide chiral 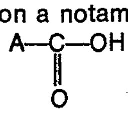 est l'acide 2,2-diméthyl 3R-(2-méthyl 1-propényl) cyclopropane-1R-carboxylique.

Dans le procédé selon l'invention, l'halogénure de bore peut être, notamment, le tribromure de bore ou le trichlorure de bore.

Selon un mode opératoire préféré du procédé de l'invention, la réaction avec l'halogénure de bore est effectuée dans le chlorure de méthylène à $-20\,°C \pm 20\,°C$.

Dans le procédé selon l'invention, on utilise de préférence 2 moles ou plus de l'halogénure de bore par mole d'ester I.

Le procédé de l'invention permet, grâce aux conditions très douces de scission de l'ester, d'obtenir avec un rendement élevé, une alléthrolone pure, exempte des produits secondaires qui peuvent se former dans les méthodes classiques d'obtention d'alléthrolone optiquement active.

Les méthodes classiques d'obtention d'alcools optiquement actifs, en général, comportent comme stade final, une hydrolyse en milieu acide ou basique, ayant pour but de libérer l'alcool optiquement actif.

En milieu basique (cf. par exemple, LA FORGE, J. Am. Chem. Soc. *74* (1952), page 5392), on pouvait craindre, à priori, que par action d'un agent basique sur l'alléthrolone, prennent naissance des réactions secondaires, telles que celles qui conduisent aux « dimères » d'alléthrolone :

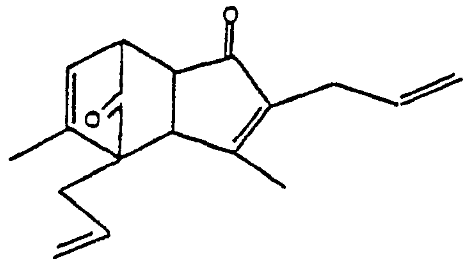

et

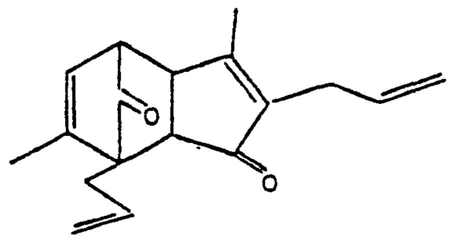

En milieu acide, on pouvait également craindre l'existence de réactions secondaires dues à la protonation aisée de l'oxygène alcoolique allylique de l'alléthrolone particulièrement réactif, pouvant donner lieu à une suite de réactions du type suivant :

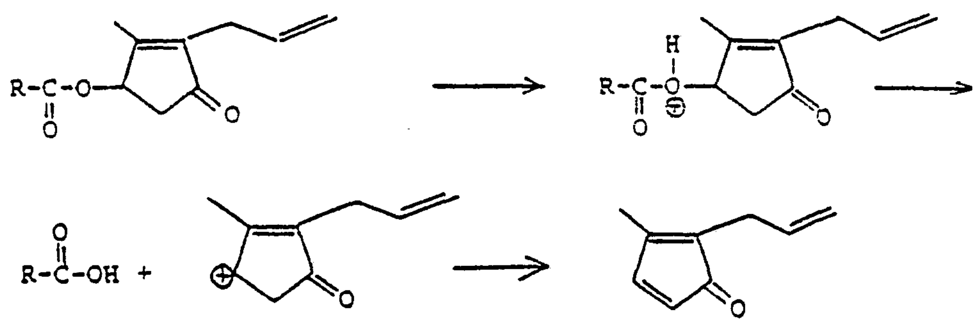

conduisant à un composé diénique lui-même très réactif.

L'exemple : (4S)2-allyl 3-méthyl 4-hydroxy 2-cyclopenten-1-one à partir de 2,2-diméthyl 3R-(2-méthyl 1-propényl) cyclopropane-1R-carboxylate de (4S) 2-allyl 3-méthyl 2-cyclopenten-1-one 4-yle.

Dans 15 ml de chlorure de méthylène, on introduit 1,5 g de 2,2-diméthyl 3R-(2-méthyl 1-propényl) cyclopropane-1R-carboxylate de (4S)2-allyl 3-méthyl 2-cyclopenten-1-one 4-yle, introduit lentement à − 20 °C, 7,8 ml de solution chlorométhylènique 1,6 M de trichlorure de bore, amène la température réactionnelle à − 12 °C, agite pendant 3 heures, verse le mélange réactionnel dans un mélange d'eau et de glace, agite pendant 15 heures, amène le pH à 10 par addition de soude diluée, sature la phase aqueuse avec du chlorure de sodium, extrait au chlorure de méthylène, sèche la phase organique, concentre à sec, chromatographie le résidu sur gel de silice en éluant par un mélange de benzène et d'acétate d'éthyle (7-3) et obtient 530 mg de (4S)2-allyl 3-méthyl 4-hydroxy 2-cyclopenten-1-one.
$/\alpha/^{20}_D = +5°$ (c = 1,2 % benzène)
Spectre U.V. (éthanol)
Maximum à 230 nm $E^1_1 = 810$, maximum à 308 nm $E^1_1 = 4$.
Dichroïsme circulaire (dioxane)
$\Delta\varepsilon = -18{,}6$ à 231 nm (maxi) ; $\Delta\varepsilon = +3{,}19$ à 322 nm (maxi)
$\Delta\varepsilon = +2{,}93$ à 332 nm (maxi).

**Revendications**

1. Procédé de préparation de (R) ou (S)2-allyl 3-méthyl 4-hydroxy 2-cyclopenten-1-one, caractérisé en ce que l'on fait réagir un halogénure de bore sur un ester d'acide carboxylique chiral $A-\underset{\underset{O}{\|}}{C}-OH$ et de (R) ou de (S) 2-allyl 3-méthyl 4-hydroxy 2-cyclopenten-1-one, de formule (I) :

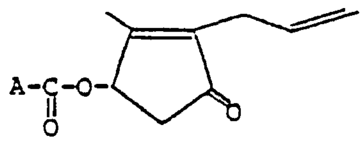

(I)

dans laquelle A représente le reste organique de l'acide chiral carboxylique $A-\underset{\underset{O}{\|}}{C}-OH$, soumet le mélange réactionnel résultant à l'action de l'eau pour obtenir avec rétention de configuration par rapport à celle existant dans l'ester de départ, respectivement la (R) ou la (S)2-allyl 3-méthyl 4-hydroxy 2-cyclopenten-1-one et l'acide chiral.

2. Procédé selon la revendication 1, caractérisé en ce que l'acide chiral $A-\underset{\underset{O}{\|}}{C}-OH$ est un acide aliphatique.

3. Procédé selon la revendication 1, caractérisé en ce que l'acide chiral $A-\underset{\underset{O}{\|}}{C}-OH$ comporte un ou plusieurs cycles.

4. Procédé selon la revendication 1 ou 3, caractérisé en ce que l'acide chiral $A-\underset{\underset{O}{\|}}{C}-OH$ est un acide de structure polycyclanique.

5. Procédé selon la revendication 1 ou 3, caractérisé en ce que l'acide chiral $A-\underset{\underset{O}{\|}}{C}-OH$ est un acide de structure stéroïde.

6. Procédé selon la revendication 1 ou 3, caractérisé en ce que l'acide chiral $A-\underset{\underset{O}{\|}}{C}-OH$ est un acide cyclopropane carboxylique.

7. Procédé selon la revendication 1 ou 6, caractérisé en ce que l'acide chiral $A-\underset{\underset{O}{\|}}{C}-OH$ est un acide carboxylique choisi dans le groupe constitué par les acides dont les noms suivent :

— les acides 2,2-diméthyl 3-(2-méthyl 1-propényl) cyclopropane-1-carboxyliques,
— les acides 2,2-diméthyl 3-(2,2-dibromovinyl) cyclopropane-1-carboxyliques,
— les acides 2,2-diméthyl 3-(2,2-dichlorovinyl) cyclopropane-1-carboxyliques,
— les acides 2,2-diméthyl 3-(cyclobutylidène méthyl) cyclopropane-1-carboxyliques,
— les acides 2,2-diméthyl 3-(cyclopentylidène méthyl) cyclopropane-1-carboxyliques,
— les acides 2,2-diméthyl 3-(fluorénylidène méthyl) cyclopropane-1-carboxyliques,
— les acides 2,2-diméthyl 3-(2-oxo 3-oxa cyclopentylidène méthyl) cyclopropane-1-carboxyliques,
— les acides 2,2-diméthyl 3-(2,2-difluorovinyl) cyclopropane-1-carboxyliques
— les acides 2-(3-chlorophényl) 2-isopropyl acétiques.

8. Procédé selon l'une quelconque des revendications 1, 6 et 7, caractérisé en ce que l'acide chiral $A-\underset{\underset{O}{\|}}{C}-OH$ est l'acide 2,2-diméthyl 3R-(2-méthyl 1-propényl) cyclopropane-1R-carboxylique.

9. Procédé selon l'une quelconque des revendications 1 à 8, caractérisé en ce que l'halogénure de bore est le tribromure de bore.

10. Procédé selon l'une quelconque des revendications 1 à 8, caractérisé en ce que l'halogénure de bore est le trichlorure de bore.

11. Procédé selon l'une quelconque des revendications 1 à 10, caractérisé en ce que la réaction avec l'halogénure de bore est effectuée dans le chlorure de méthylène à − 20 °C ± 20 °C.

12. Procédé selon l'une quelconque des revendications 1 à 11, caractérisé en ce que l'on utilise 2 moles ou plus de l'halogénure de bore par mole d'ester I.

**Claims**

1. Process for preparing (R) or (S) 2-allyl 3-methyl 4-hydroxy 2-cyclopenten-1-one, characterized in that a boron halide is reacted with an ester of a chiral carboxylic acid $A-\underset{\underset{O}{\|}}{C}-OH$ and of (R) or (S) 2-allyl 3 3-methyl 4-hydroxy 2-cyclopenten-1-one, of formula (I) :

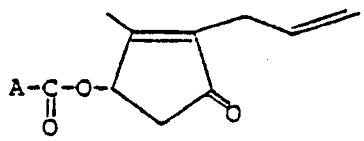

(I)

in which A represents the organic residue of the chiral carboxylic acid $A-\underset{\underset{O}{\|}}{C}-OH$, and the resulting reaction mixture is subjected to the action of water to obtain, with retention of configuration with respect to that existing in the starting ester, respectively (R) or (S) 2-allyl 3-methyl 4-hydroxy 2-cyclopenten-1-one and the chiral acid.

2. Process according to claim 1, characterized in that the chiral acid $A-\underset{\underset{O}{\|}}{C}-OH$ is an aliphatic acid.

3. Process according to claim 1, characterized in that the chiral acid $A-\underset{\underset{O}{\|}}{C}-OH$ contains one or more rings.

4. Process according to claim 1 or 3, characterized in that the chiral acid $A-\underset{\underset{O}{\|}}{C}-OH$ is an acid of polycyclanic structure.

5. Process according to claim 1 or 3, characterized in that the chiral acid $A-\underset{\underset{O}{\|}}{C}-OH$ is an acid of steroid structure.

6. Process according to claim 1 or 3, characterized in that the chiral acid $A-\underset{\underset{O}{\|}}{C}-OH$ is a cyclopropane carboxylic acid.

7. Process according to claim 1 or 6, characterized in that the chiral acid $A-\underset{\underset{O}{\|}}{C}-OH$ is a carboxylic acid selected from the group constituted by the chiral acids of which the names are as follows :

— the 2,2-dimethyl 3-(2-methyl 1-propenyl) cyclopropane-1-carboxylic acids,
— the 2,2-dimethyl 3-(2,2-dibromovinyl) cyclopropane-1-carboxylic acids,
— the 2,2-dimethyl 3-(2,2-dichlorovinyl) cyclopropane-1-carboxylic acids,
— the 2,2-dimethyl 3-(cyclobutylidene methyl) cyclopropane-1-carboxylic acids,
— the 2,2-dimethyl 3-(cyclopentylidene methyl) cyclopropane-1-carboxylic acids,
— the 2,2-dimethyl 3-(fluorenylidene methyl) cyclopropane-1-carboxylic acids,
— the 2,2-dimethyl 3-(2-oxo 3-oxa cyclopentylidene methyl) cyclopropane-1-carboxylic acids,
— the 2,2-dimethyl 3-(2,2-difluorovinyl) cyclopropane-1-carboxylic acids and the 2-(3-chlorophenyl) 2-isopropyl acetic acids.

8. Process according to any one of claims 1, 6 and 7, characterized in that the chiral acid $A-\underset{\underset{O}{\|}}{C}-OH$ is 2,2-dimethyl 3R-(2-methyl 1-propenyl) cyclopropane-1R-carboxylic acid.

9. Process according to any one of claims 1 to 8, characterized in that the boron halide is boron tribromide.

10. Process according to any one of claims 1 to 8, characterized in that the boron halide is boron trichloride.

11. Process according to any one of claims 1 to 10, characterized in that the reaction with the boron halide is carried out in methylene chloride at – 20 °C ± 20 °C.

12. Process according to any one of claims 1 to 11, characterized in that two or more moles of the boron halide are used per mole of ester I.

**Ansprüche**

1. Verfahren zur Herstellung von (R)- oder (S)-2-Allyl-3-methyl-4-hydroxy-2-cyclopenten-1-on, dadurch gekennzeichnet, daß man ein Borhalogenid mit einem Ester der chiralen Carbonsäure $A-\underset{\underset{O}{\|}}{C}-OH$ und des (R)- oder (S)-2-Allyl-3-methyl-4-hydroxy-2-cyclopenten-1-ons der Formel (I) :

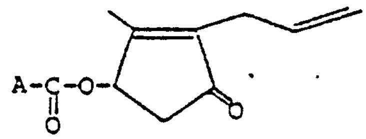

(I)

worin A den organischen Rest der chiralen Carbonsäure $A{-}\underset{\underset{O}{\|}}{C}{-}OH$ bedeutet, umsetzt und das erhaltene Reaktionsgemisch der Einwirkung von Wasser unterzieht, um unter Beibehaltung der Konfiguration in bezug auf die in dem Ausgangsester vorliegende das (R)- bzw. (S)-2-Allyl-3-methyl-4-hydroxy-2-cyclopenten-1-on und die chirale Säure zu erhalten.

2. Verfahren gemäß Anspruch 1, dadurch gekennzeichnet, daß die chirale Säure $A{-}\underset{\underset{O}{\|}}{C}{-}OH$ eine aliphatische Säure ist.

3. Verfahren gemäß Anspruch 1, dadurch gekennzeichnet, daß die chirale Säure $A{-}\underset{\underset{O}{\|}}{C}{-}OH$ einen oder mehrere Ringe enthält.

4. Verfahren gemäß Anspruch 1 oder 3, dadurch gekennzeichnet, daß die chirale Säure $A{-}\underset{\underset{O}{\|}}{C}{-}OH$ eine Säure mit Polycyclanstruktur ist.

5. Verfahren gemäß Anspruch 1 oder 3, dadurch gekennzeichnet, daß die chirale Säure $A{-}\underset{\underset{O}{\|}}{C}{-}OH$ eine Säure mit Steroidstruktur ist.

6. Verfahren gemäß Anspruch 1 oder 3, dadurch gekennzeichnet, daß die chirale Säure $A{-}\underset{\underset{O}{\|}}{C}{-}OH$ eine Cyclopropancarbonsäure ist.

7. Verfahren gemäß Anspruch 1 oder 6, dadurch gekennzeichnet, daß die chirale Säure $A{-}\underset{\underset{O}{\|}}{C}{-}OH$ eine Carbonsäure, ausgewählt aus der Gruppe der chiralen Säuren mit den folgenden Bezeichnungen, ist :

— den 2,2-Dimethyl-3-(2-methyl-1-propenyl)-cyclopropane-1-carbonsäuren,
— den 2,2-Dimethyl-3-(2,2-dibromvinyl)-cyclopropan-1-carbonsäuren,
— den 2,2-Dimethyl-3-(2,2-dichlorvinyl)-cyclopropan-1-carbonsäuren,
— den 2,2-Dimethyl-3-(cyclobutylidenmethyl)-cyclopropan-1-carbonsäuren,
— den 2,2-Dimethyl-3-(cyclopentylidenmethyl)-cyclopropan-1-carbonsäuren,
— den 2,2-Dimethyl-3-(fluorenylidenmethyl)-cyclopropan-1-carbonsäuren,
— den 2,2-Dimethyl-3-(2-oxo-3-oxa-cyclopentylidenmethyl)-cyclopropan-1-carbonsäuren,
— den 2,2-Dimethyl-3-(2,2-difluorvinyl)-cyclopropan-1-carbonsäuren und
— den 2-(3-Chlorphenyl)-2-isopropyl-essigsäuren.

8. Verfahren gemäß einem der Ansprüche 1, 6 und 7, dadurch gekennzeichnet, daß die chirale Säure $A{-}\underset{\underset{O}{\|}}{C}{-}OH$ die 2,2-Dimethyl-3R-(2-methyl-1-propenyl)-cyclopropan-1R-carbonsäure ist.

9. Verfahren gemäß einem der Ansprüche 1 bis 8, dadurch gekennzeichnet, daß das Borhalogenid Bortribromid ist.

10. Verfahren gemäß einem der Ansprüche 1 bis 8, dadurch gekennzeichnet, daß das Borhalogenid Bortrichlorid ist.

11. Verfahren gemäß einem der Ansprüche 1 bis 10, dadurch gekennzeichnet, daß die Umsetzung mit dem Borhalogenid in Methylenchlorid bei $-20\,°C \pm 20\,°C$ durchgeführt wird.

12. Verfahren gemäß einem der Ansprüche 1 bis 11, dadurch gekennzeichnet, daß man 2 Mol oder mehr Borhalogenid je Mol Ester I verwendet.